(19) Europäisches Patentamt

European Patent Office

Office européen des brevets

(11) Publication number: **0 639 647 A2**

# EUROPEAN PATENT APPLICATION

(21) Application number: **94110526.4**

(22) Date of filing: **06.07.94**

(51) Int. Cl.6: **C12Q 1/68**

(30) Priority: **08.07.93 JP 168895/93**

(43) Date of publication of application:
**22.02.95 Bulletin 95/08**

(84) Designated Contracting States:
**DE FR GB IT**

(71) Applicant: **TANABE SEIYAKU CO., LTD.**
**2-10, Dosho-machi 3-chome**
**Chuo-ku**
**Osaka (JP)**
Applicant: **EIKEN CHEMICAL Co., Ltd.**
**33-8, Hongo 1-chome**
**Bunkyo-ku,**
**Tokyo (JP)**

(72) Inventor: **Yamagata, Koichi**
**No. 14-17, Oike-Miyamadai,**
**Kita-ku**
**Kobe-shi,**
**Hyogo-ken (JP)**
Inventor: **Umemura, Isao**
**No. 3-8-43, Koyodai**
**Kawanishi-shi,**
**Hyogo-ken (JP)**
Inventor: **Shibatani, Takeji**
**No. 3-18-5, Uzumoridai,**
**Higashinada-ku**
**Kobe-shi,**
**Hyogo-ken (JP)**

(74) Representative: **Hansen, Bernd, Dr.**
**Dipl.-Chem. et al**
**Hoffmann, Eitle & Partner,**
**Patentanwälte,**
**Arabellastrasse 4**
**D-81925 München (DE)**

(54) **Assay for detecting nucleic acid sequence.**

(57) Assay for detecting nucleic acid sequence, which comprises the steps: [A] contacting (1) a target single-stranded nucleic acid, (2) a pair of nucleic acid primers having nucleotide sequence complementary to the target nucleic acid and its complementary chain respectively, (3) a fluorescent-labeled nucleic acid probe which has a nucleotide sequence designed so as to be hybridized to the target nucleic acid downstream (at the side of 3'-terminus) from where the nucleic acid primer is hybridized and has a modified 3'-end so that the nucleotide chain is not elongated by a nucleic acid polymerase, and (4) a nucleic acid polymerase having exonuclease activity specific to a double-stranded nucleic acid in the presence of substrates for the enzyme (four kinds of nucleoside triphosphate), and thereby elongating the said nucleic acid primer chain and simultaneously hydrolyzing only the fluorescent-labeled nucleic acid probe hybridized to the target nucleic acid; [B] denaturing the chain-elongated product of primer to a single-stranded form; [C] amplifying the target nucleic acid by repeating the steps A and B; and [D] determining the change in fluorescence polarization resulted by the hydrolysis of the probe. This method is effective to detect the target nucleic acid sequence more simply with higher sensitivity and higher reliability in comparison with known methods, and further has advantages that because of no carryover or contamination of the amplified nucleic acids into subsequent reactions, undesirable false positive result can be avoided.

EP 0 639 647 A2

This invention relates to an assay method for detecting a nucleic acid sequence, which is useful for diagnosis of infectious diseases caused by microorganisms such as bacteria, virus and the like.

Relevant Art

In diagnosis of diseases induced by change of gene such as hereditary diseases, or diseases induced by exogenous gene such as virus DNA, it is useful to detect directly the abnormal gene or the exogenous gene in addition to the clinical observation of the patients, biochemical test or culture test of the sample obtained from the patients. Direct detection of DNAs or RNAs having specific nucleotide sequence become possible owing to the recent establishment of genetic engineering technique which has comparatively good reproducibility and also the development of various nucleic acid probes, and it has been studied to apply such a direct detection of DNAs or RNAs to clinical diagnosis of diseases.

The assay for the presence of a gene having a specific nucleotide sequence in a test sample has hitherto been done by a dot blotting method which comprises denaturing an extracted polynucleotide component to a single-stranded form by treating it with an alkali (e.g. sodium hydroxide) or by subjecting it to heat treatment, immobilizing the resultant onto a nitrocellulose membrane, hybridizing it with a nucleic acid probe labeled with a marker such as a radioactive isotope (RI), biotin, etc. and then detecting the hybridized product. However, said method requires very complicated procedures such as immobilization of the polynucleotide component, removal of unhybridized probe (B/F separation) after the hybridization, and hence, it is very difficult to treat a plenty of test samples at one time.

Recently, some improved methods eliminating the above problems have been proposed, for example, a method comprising hybridizing the target DNA with a fluorescent-labeled DNA probe and determining the change in fluorescence polarization (i.e. increase in the fluorescence anisotropy) owing to increase in apparent molecular weight (cf. Japanese Patent First Publication (Kokai) No. 75958/1990), and a method comprising treating a double-stranded DNA formed from a fluorescent-labeled DNA probe and a target DNA with a λ-exonuclease to hydrolyze only the hybridized probe and determining the change in fluorescence polarization owing to decrease in molecular weight of the labeled probe (cf. International Patent Publication WO91/17264). However, these methods have still a problem of less sensitivity which causes false negative data, and hence, they have insufficient reliability.

In order to avoid such a false negative result owing to insufficient sensitivity, it has also been proposed to amplify the target gene contained in the test sample by polymerase chain reaction (PCR) method, and the like. This PCR method has generally been employed for *in vitro* amplification of nucleic acid, but it has a defect that it amplifies sometimes also nucleic acid having nucleotide sequence different from the target gene. Accordingly, it is necessary to check whether the target gene is amplified or not by transferring a part of the reaction mixture and testing with using the nucleic acid probe, etc. thereof by hybridization method, which requires complicated procedure such as immobilization of the amplified gene. Moreover, it tends to occur contamination of the amplified nucleic acid sequence due to diffusion, and hence, undesirable false positive appears disadvantageously frequently. This is particularly serious problem in case of detection of a plenty of test samples at one time. From this viewpoint, the assay method for the detection of nucleic acid using PCR method has still not practically been employed in clinical test.

Thus, it has been desired to develop an improved assay method for detecting nucleic acid components having the target nucleotide sequence, which has high sensitivity, high reliability and is easily practiced with a simple procedure.

The present inventors have intensively studied to find the desired improved assay method for the detection of nucleic acid sequence and have newly found that when the polymerase chain reaction (PCR) is carried out with a nucleic acid polymerase having also an exonuclease activity specific to a double-stranded nucleic acid in the presence of a fluorescent-labeled nucleic acid probe having a chemically modified 3'-end so that no elongation of the nucleotide chain is induced by said nucleic acid polymerase, the above probe is hydrolysed by said enzyme only when the target nucleic acid is amplified, and hence, the desired determination of the target nucleic acid can directly and simply be done by determining the change in fluorescence polarization (decrease in the fluorescence anisotropy) of the test sample containing said hydrolysed product without necessity of transferring the PCR reaction mixture from the reaction vessel. Thus, the present invention has been accomplished.

Brief Description of the Invention

An object of this invention is to provide an improved assay method for detecting a nucleic acid sequence in a test sample with a simple procedure and with high reliability. This and other objects of the

2

invention will be apparent from the following description.

Detailed Description of the Invention

This invention provides an assay method for detecting nucleic acid sequence, which comprises the steps:

[A] contacting (1) a target single-stranded nucleic acid, (2) a pair of nucleic acid primers having nucleotide sequence complementary to the target nucleic acid and its complementary chain respectively, (3) a fluorescent-labeled nucleic acid probe which has a nucleotide sequence designed so as to be hybridized to the target nucleic acid downstream (at the side of 3'-terminus) from where the nucleic acid primer is hybridized and has a modified 3'-end so that the nucleotide chain is not elongated by a nucleic acid polymerase, and (4) a nucleic acid polymerase having also an exonuclease activity specific to a double-stranded nucleic acid in the presence of four kinds of nucleoside triphosphate which are a substrate for the enzyme, and thereby elongating the said nucleic acid primer chain, and simultaneously hydrolyzing only the fluorescent-labeled nucleic acid probe hybridized to the target nucleic acid by the exonuclease activity of said nucleic acid polymerase,

[B] denaturing the chain-elongated product of primer to a single-stranded nucleic acid,

[C] amplifying the target nucleic acid by repeating the above steps [A] and [B] the desired times,

[D] determining the change in fluorescence polarization resulted by the hydrolysis of the fluorescent-labeled nucleic acid probe.

The target nucleic acid used in this invention is not specified but includes any nucleic acids which nucleotide sequence is known, and both of DNA and RNA are inclusive. Moreover, the target nucleic acid is not limited by the origin but includes any nucleic acid originated from eukaryotic organisms, prokaryotic organisms, and viruses. Besides, when the nucleic acid to be detected is RNA, if desired, a cDNA derived from the RNA by using a reverse transcriptase may be used as the target nucleic acid.

The nucleic acid primer and the nucleic acid probe used in this invention may be either DNA or RNA. Among them, preferred examples of said probe include probes having such a nucleotide sequence as to hybridize specifically and stably to the target nucleic acid and its complementary chain depending on the kinds of the target nucleic acid and its complementary chain (i.e. depending on nucleotide sequence). Under taking the specificity in the hybridization into consideration, the length of the nucleotide sequence of said primer and probe is preferably 15 mer or more, more preferably in the range of 20 to 30 mer. Any known nucleic acid primer and probe having such a nucleotide sequence can be used in this invention, and further, any nucleic acid probe having a newly designed nucleotide sequence is also usable in this invention. Besides, the nucleic acid probe is designed so as to be hybridized to the target nucleic acid downstream (at the side of 3'-terminus) from where the above nucleic acid primer is hybridized and has a chemically modified 3'-end so that no elongation of the nucleotide chain is induced by a nucleic acid polymerase. Particularly preferred probe is hardly hydrolyzed by non-specific hydrolysis. The modification at 3'-terminus of the nucleic acid probe is effected by phosphating the 3'-OH group or by adding thereto a few bases which are not hybridized to the target nucleic acid (i.e., mismatching bases).

The nucleic acid primer and probe can be prepared by known methods, for example, by a chemical synthetic method, or by cutting out a fragment having the desired nucleotide sequence (oligonucleotide) from a genome with an appropriate restriction enzyme. The fluorescent-labeled nucleic acid probe can be prepared by labeling the oligonucleotide as prepared above with a fluorescent agent by a conventional method. The fluorescent agent to be used for the labeling includes, for example, fluorescein derivatives such as fluorescein isothiocyanate (FITC), rhodamine isothiocyanate (RITC), tetramethylrhodamine isothiocyanate (TRITC), eosine isothiocyanate (EITC), and the like, particularly preferably FITC.

The labeling method of the nucleic acid probe with the fluorescent agent can be carried out by a known method, for example, by labeling directly the base of the nucleic acid with an amino group-introducing agent [e.g. 2-(4-monomethoxytritylamino)ethyl)-(2-cyanoethyl)-N,N-diisopropyl)phosphoramidite, etc.], a thiol group-introducing agent [e.g. S-(trityl-2-mercaptoethyl)-(2-cyanoethyl)-(N,N-diisopropyl)phosphoramidite, (S-trityl-6-mercaptohexyl)-(2-cyanoethyl)-N,N-diisopropyl)phosphoramidite, etc.]; by substituting a phosphoric acid moiety at the site to be labeled in the nucleic acid for a phosphonic acid moiety having a functional group (i.e., introduction of linker) and then binding the fluorescent agent to the probe via said functional group (cf. Japanese Patent First Publication (Kokai) No. 44353/1986).

The nucleic acid polymerase used in this invention has also an exonuclease activity specific to a double-stranded nucleic acid. Suitable examples of the nucleic acid polymerase are Taq DNA polymerase produced by *Thermus aquaticus*, DNA polymerase I produced by *Escherichia coli*, among which the particularly preferred one is Taq DNA polymerase which is heat resistant and is stable even at a high temperature.

Taq DNA polymerase originated form *Thermus aquaticus* is disclosed in Proc. Natl. Acad. Sci. USA. vol. 88, pp. 7276-7280, August 1991. DNA Polymerase I produced by *Escherichia coli* is commercially available, for example, DNA Polymerase I, Code No. DPL-101 and DPL-102 (manufactured by TOYOBO CO., LTD.).

Four kinds of nucleoside triphosphate used as a substrate for the nucleic acid polymerase include deoxyadenosine triphosphate (dATP), deoxyguanosine triphosphate (dGTP), deoxycytidine triphosphate (dCTP), and deoxythymidine triphosphate (dTTP).

Each step in the assay method of this invention is carried out as follows.

[Step A]

This step comprises (i) hybridization (annealing) reaction of the nucleic acid primers and the nucleic acid probes to the target nucleic acid and its complementary chain, and (ii) a chain-elongation reaction of the primer and hydrolysis of the probe by a nucleic acid polymerase, and these reaction can be carried out in an aqueous solution containing an appropriate buffer (e.g. Tris-HCl buffer). This hybridization reaction and polymeraze reaction can usually be carried out at a temperature of 55°C to 75°C.

[Step B]

The denaturation of the chain-elongated product of primer to a single-stranded nucleic acid can be carried out, for example, by subjecting a solution containing the chain-elongated product of primer to heat treatment at 94°C to 95°C for 0.5 - 1 minute.

[Step C]

The amplification of the target nucleic acid may be carried out by repeating the above steps [A] and [B] under the same conditions a plural time until the desired amount of the target nucleic acid is obtained. When the steps [A] and [B] are repeated "n" times, the target nucleic acid is amplified in $2^{n-1}$-folds theoretically.

The above steps A, B and C can be easily and efficiently carried out with a commercially available apparatus for the PCR (polymerase chain reaction).

[Step D]

The change in fluorescence polarization resulted by the hydrolysis of the fluorescent-labeled nucleic acid probe may be determined by measuring the fluorescence polarization of each of the sample solution obtained through the procedure (the steps A, B and C) and of a solution containing no target nucleic acid (control solution), for example, by a similar method disclosed in Analytica Chimica Acta, Vol. 244, pages 207-213 (1991), and comparing the fluorescence anisotropy calculated from the resulting data in accordance with the following formula:

$$\text{Fluorescence anisotropy } (r) = \frac{[I_{VV} - I_{VH} \times G]}{[I_{VV} + 2 I_{VH} \times G]} \quad (I)$$

wherein G is a correction factor showing a conversion efficiency peculiar to the apparatus and is expressed by $G = I_{HV}/I_{HH}$, $I_{VV}$ means a fluorescence intensity when the apparatus is set so that the polarizing plates at the exciting region and at the detecting region can transmit a vertically polarized light; $I_{VH}$ means a fluorescence intensity when the apparatus is set so that the polaring plate at the exciting region can transmit a vertically polarized light and the polarizing plate at the detecting region can transmit a horizontally polarized light; $I_{HV}$ means a fluorescence intensity when the apparatus is set so that the polarizing plate at the exciting region can transmit a horizontally polarized light and the polarizing plate at the detecting region can transmit a vertically polarized light; and $I_{HH}$ means a fluorescence intensity when the apparatus is set so that the polarizing plates at the exciting region and at the detecting region can transmit a horizontally polarized light.

4

Alternatively, the change in fluorescence polarization may be determined with lapse of time without using any control solution by measuring the fluorescence polarization of each solution in the step of amplification of the target gene (step C) at appropriate intervals.

The excitation wavelength and the emission wavelength to be used in the measurement of fluorescence polarization of the sample solution are variable depending on the kinds of the fluorescent agent employed. For example, when fluorescein isothiocyanate is used as the fluorescent agent, the excitation wavelength and the emission wavelength are 490 nm and 520 nm, respectively.

The above measurement of the fluorescence polarization can be done with neither transferring the sample solution from the reaction vessel nor diluting the sample solution, unless it is required.

According to the assay method of this invention, the nucleic acid having target nucleotide sequence can specifically be detected, and hence, it can be applied to the detection of microbial nucleic acids for diagnosis of various infectious diseases. For example, the diagnosis of infectious diseases of viruses such as hepatitis C virus (HCV), cytomegalovirus (CMV), Epstein-Barr virus (EBV), herpes virus, human immunodeficiency virus (HIV), etc.; bacteria such as *Escherichia coli*, *Mycobacterium tuberculosis*, Typhoid bacillus, *Salmonella*, *Vibrio parahaemolyticus*, etc.; or *Mycoplasma* can be done by using a nucleic acid primer and a nucleic acid probe which are designed so as to be able to recognize the nucleotide sequences peculiar to DNA or RNA originated from the above microorganisms.

Many examples of the nucleic acid primer and the nucleic acid probe useful for the detection of nucleic acids of the above-mentioned microorganisms are known, for example, a nucleic acid probe for the detection of HCV as disclosed in Japanese Patent First Publication (Kokai) No. 103180/1991, a nucleic acid probe for the detection of EBV as disclosed in Japanese Patent First Publication (Kokai) No. 3800/1993, nucleic acid primer and nucleic acid probe for the detection of *Vibrio parahaemolyticus* as disclosed in Japanese Patent First Publication (Kokai) No. 20299/1992, nucleic acid primer and nucleic acid probe for the detection of *Mycoplasma* as disclosed in Japanese Patent First Publication (Kokai) No. 88/1993, but they are not limited thereto.

Throughout the description and claims, DNA means deoxyribonucleic acid, RNA means ribonucleic acid, A means adenine, C means cytosine, T means thymidine, and G means guanine, unless specified otherwise.

Examples

This invention is illustrated by the following examples but should not be construed to be limited thereto.

Example 1

[Experimental method]

(1) Preparation of a fluorescent-labeled nucleic acid probe:

Thirty-mer oligo DNA having the following nucleotide sequence complementary to the region of the base number 5508 - 5537 of M13mp18 phage DNA (cf. Gene, Vol. 33, pages 103-119, 1985) which is the target DNA:

5'-TGCGCGTAACCACCACACCCGCCGCGCTTAp

wherein p means 3'-phosphoryl residue, was synthesized by using a DNA synthesizer (Type 381, manufactured by Applied Biosystems). In this synthesis, a linker was introduced into the 5'-terminal base (T) of the oligonucleotide in order to bind thereto a fluorescent agent (fluorescein isothiocyanate, abbreviated hereinafter as "FITC") by using Amino-Modifier-dT (i.e. 5'-dimethoxytrityl-5-[N-trifluoroacetylaminohexyl)-3-acrylimido]-2'-deoxyuridine-3'-[(2-cyanoethyl)-(N,N-diisopropyl)]phosphoramidite, manufactured by Glen Research). Besides, the 3'-terminal OH group of the above oligo DNA was phosphorylated by using 2-[2-(4,4'-dimethoxytrityloxy)ethylsulfonyl]ethyl-(2-cyanoethyl)-(N,N-diisopropyl)phosphoramidite, manufactured by Glen Research). The oligo DNA was labeled with FITC according to the method disclosed in Nucleic Acid Research, Vol. 19, pages 4097-4102 (1991) as follows.

The above-prepared linker-introduced oligo DNA (20 nmol) was dried and dissolved in a 20 mM FITC solution (0.1 ml, a mixture of 200 mM carbonate buffer (pH 9) and dimethylformamide in a ratio of 6 : 4), and the mixture was reacted at 25°C for 48 hours.

After the reaction, the FITC-labeled DNA probe was isolated from the reaction mixture with a reversed-phase high-performance liquid chromatography (column, YMC-Pack ODS-AM (250 x 6.0 mm), manufactured by YMC Co.). The desired FITC-labeled DNA prove was identified by measuring the ultraviolet-visible absorption spectrum (240 - 600 nm) of each fraction thus obtained.

(2) Detection of the target nucleic acid:

By using the fluorescent-labeled nucleic acid probe prepared above (1), the detection of the target nucleic acid was carried out as follows.

There was prepared a sample solution (200 $\mu$l) containing 50 mM potassium chloride, 10 mM Tris-HCl buffer (pH 8.3), 20 pmol oligonucleotide primers (5'-CCGATAGTTTGAGTT CTTCTACTCAGGC-3' and 5'-GAAGAAAGCGAAAGGAGCGGGCGCTAGGGC-3', SEQ ID NO: 2 and NO: 3), 200 $\mu$M of each dNTP (dATP, dCTP, dGTP and dTTP), 3 mM magnesium chloride, 2.5 Unit of thermostable DNA polymerase (Ampli Taq DNA Polymerase, manufactured by Perkin Ermer Cetus), 20 pmol fluorescent-labeled nucleic acid probe, and a target DNA (single-stranded M13mp18 DNA, $10^6$ molecules). The half (100 $\mu$l) of this sample solution was diluted with 0.1 M Tris-HCl buffer (pH 8.0) containing 20 mM EDTA to 400 $\mu$l volume, and the fluorescence polarization of the solution was measured at 25°C with a fluorescence spectrophotometer equipped with a set of polarizing plates (Shimadzu RF-5000, manufactured by Shimadzu Co.). Based on the resulting data, the fluorescence anisotropy ($r_1$) was calculated in accordance with the formula (I) disclosed hereinbefore.

Thereafter, the remaining sample solution (100 $\mu$l) was layered with one pellet of a wax (Ampliwax PCR Gem, manufactured by Perkin Elmer Cetus), and then, it was subjected to 40 cycles of denaturation with heating (95°C for 1 minute), and annealing and elongation of primer chain (60°C for 1 minute) with a PCR apparatus (Type PC-700, manufactured by Astec Co.), and thereafter, it was subjected to chain-elongation reaction at 60°C for 10 minutes. The reaction mixture was diluted with 0.1 M Tris-HCl buffer (pH 8.0) containing 20 mM EDTA to 400 $\mu$l volume, and then the fluorescence anisotropy ($r_2$) was determined by measuring the fluorescence polarization in the same manner as described above.

Besides, as a control, a sample solution containing no target DNA (Control 1) and a sample solution containing a λDNA and DNAs of SEQ ID NO: 4 and NO: 5 (primer DNAs having nucleotide sequence complementary to the λDNA) instead of the target DNA and DNAs of SEQ ID NO: 2 and NO: 3, respectively (Control 2) were treated in the same manner as described above, and then, the fluorescence anisotropy ($r_1$ and $r_2$) were measured likewise.

[Results]

The results are shown in Table 1.

Table 1

| | Fluorescence anisotropy | |
|---|---|---|
| | $r_1$ | $r_2$ |
| M13mp18 DNA (Method of this invention) | 70 | 50 |
| No DNA (Control 1) | 67 | 65 |
| λDNA (Control 2) | 67 | 67 |
| (Note: the "r" value is expressed by 1000-folds of the calculated data) | | |

[Study of the Results]

As is clear from the above results, by carrying out the PCR in the presence of a fluorescent-labeled nucleic acid probe, only when the desired target DNA was amplified, the hydrolysis of the fluorescent-labeled nucleic acid probe and the decrease in the molecular weight of the probe thereby occurred and as a result the fluorescence anisotropy (r) lowered. Thus, the target DNA can specifically be detected by carrying out the PCR in the presence of a fluorescent-labeled nucleic acid probe having a nucleotide sequence complementary to the target DNA and then measuring the fluorescence anisotropy of the reaction mixture.

6

Besides, by analyzing the above PCR reaction mixture by electrophoresis with a 2 % agarose gel, it was confirmed that in the reaction mixture containing the target DNA (M13mp18 DNA), which is a template DNA for the PCR, the fragment of expected size (350 bp; the distance between two primers of SEQ ID NO: 2 and NO: 3) was amplified in the same degree regardless of the presence or absence of the fluorescent-labeled nucleic acid probe having a nucleotide sequence complementary to the target DNA. From this fact, it is clear that the fluorescent-labeled nucleic acid probe does not function as a primer and does not inhibit the PCR.

Moreover, even in the reaction mixture where the DNA (λDNA), which is not complementary to the fluorescent-labeled nucleic acid probe, was amplified (Control 2), the fragment of expected size (500 bp; the distance between two primers of SEQ ID NO: 4 and NO: 5) was amplified. From this fact, it is also understood that the fluorescent-labeled nucleic acid probe does not inhibit the PCR. Besides, in the reaction mixture containing a fluorescent-labeled nucleic acid probe but no target DNA (no template DNA) (Control 1), no amplification of DNA was observed.

Furthermore, by analyzing the PCR reaction mixtures obtained by the present invention and in Control 1 and Control 2 by a reversed-phase high-performance liquid chromatography (detected at the excitation wavelength = 490 nm and the emission wavelength = 520 nm), only in the reaction mixture containing the target DNA having a nucleotide sequence complementary to the fluorescent-labeled nucleic acid probe (of the present invention), there was detected the hydrolyzed product derived from the fluorescent-labeled nucleic acid probe. Thus, it is clear that only when the DNA having the target nucleotide sequence is amplified, the fluorescent-labeled nucleic acid probe is hydrolyzed.

Example 2

[Experimental method]

Test samples containing various amounts of the target DNA (single-stranded M13mp18 DNA) were prepared and treated in the same manner as described in Example 1, and the fluorescence polarization of the reaction mixtures was measured, and then the fluorescence anisotropy thereof were determined likewise.

[Results]

The results are shown in Table 2.

Table 2

| Amount of target DNA (molecules) | 0 | $10^4$ | $10^5$ | $10^6$ | $10^7$ | $10^8$ |
|---|---|---|---|---|---|---|
| Fluorescence anisotropy ($r_1$) | 70 | 69 | 71 | 70 | 70 | 70 |
| Fluorescence anisotropy ($r_2$) | 68 | 53 | 48 | 46 | 42 | 42 |
| (Note: the "r" values are expressed by 1000-folds of the calculated data) | | | | | | |

[Study of the results]

As is clear from the above results, the target DNA can be detected simply with high sensitivity by the assay method of this invention.

Effects of the Invention

The assay method of this invention can detect the target nucleic acid with higher sensitivity in comparison with the known methods, for example the method comprising determining the change in fluorescence polarization (i.e. increase in the fluorescence anisotropy) owing to increase in apparent molecular weight of DNA by the hybridization of the target DNA with a fluorescent-labeled DNA probe (cf. Japanese Patent First Publication (Kokai) No. 75958/1990), and the method comprising hydrolyzing a fluorescent-labeled DNA probe with a λ-exonuclease and determining the change in fluorescence polarization owing to decrease in molecular weight of the probe (cf. International Patent Publication WO91/17264), and hence, it has an advantage of the prevention of occurrence of false negative data. Besides, in case of the PCR alone, it has a defect that undesirable false positive data are frequently obtained due to the amplification of nucleic acid having a nucleotide sequence other than the target nucleotide sequence, but on the contrary, according to the assay method of this invention, only the nucleic acid having the target nucleotide sequence can be specifically detected among the amplified nucleic acids and thereby occurrence of the undesirable false positive result can be avoided. Thus, the assay method of this invention can give the detecting result with higher reliability.

Moreover, the assay method of this invention can be used for the detection of the target nucleic acid sequence without necessity of transferring the PCR reaction mixture from the reaction vessel. Therefore, the risk of carryover contamination of the amplified nucleic acids into subsequent reactions and hence occurrence of false positive data can also be avoided.

SEQUENCE LISTING

(1) GENERAL INFORMATION:

    (i) APPLICANT:

        (A) NAME: Tanabe Seiyaku Co., Ltd.

        (B) STREET: 2-10 Dosho-machi 3-chome, Chuo-ku

        (C) CITY: Osaka

        (E) COUNTRY: Japan

        (F) POSTAL CODE (ZIP): none


        (A) NAME: Eiken Chemical Co., Ltd.

        (B) STREET: 33-8 Hongo 1-chome, Bunkyo-ku

        (C) CITY: Tokyo

        (E) COUNTRY: Japan

        (F) POSTAL CODE (ZIP): none


    (ii) TITLE OF INVENTION: Assay for Detecting Nucleic Acid Sequence

    (iii) NUMBER OF SEQUENCES: 5

    (iv) COMPUTER READABLE FORM:

        (A) MEDIUM TYPE: Floppy disk

        (B) COMPUTER: IBM PC compatible

        (C) OPERATING SYSTEM: PC-DOS/MS-DOS

        (D) SOFTWARE: PatentIn Release #1.0, Version #1.25 (EPO)


    (vi) PRIOR APPLICATION DATA:

        (A) APPLICATION NUMBER: JP 168895/1993

        (B) FILING DATE: 08-JUL-1993

SEQ ID NO: 1

SEQUENCE LENGTH:  30

SEQUENCE TYPE:  nucleic acid

STRANDEDNESS:  single

TOPOLOGY:  linear

MOLECULE TYPE:  other nucleic acid, synthetic DNA probe

ORIGINAL SOURCE:  phage M13mp18

SEQUENCE DESCRIPTION:

     TGCGCGTAACCACCACACCCGCCGCGCTTA     30


SEQ ID NO: 2

SEQUENCE LENGTH:  28

SEQUENCE TYPE:  nucleic acid

STRANDEDNESS:  single

TOPOLOGY:  linear

MOLECULE TYPE:  other nucleic acid, synthetic DNA primer

ORIGINAL SOURCE:  phage M13mp18

SEQUENCE DESCRIPTION:

     CCGATAGTTTGAGTTCTTCTACTCAGGC     28


SEQ ID NO: 3

SEQUENCE LENGTH:  30

SEQUENCE TYPE:  nucleic acid

STRANDEDNESS:  single

TOPOLOGY:  linear

MOLECULE TYPE:  other nucleic acid, synthetic DNA primer

ORIGINAL SOURCE:  phage M13mp18

SEQUENCE DESCRIPTION:

     GAAGAAAGCGAAAGGAGCGGGCGCTAGGGC     30


SEQ ID NO: 4

SEQUENCE LENGTH:  25

SEQUENCE TYPE:  nucleic acid

```
STRANDEDNESS:  single

TOPOLOGY:  linear

MOLECULE TYPE:  other nucleic acid, synthetic DNA primer

ORIGINAL SOURCE:  λ phage

SEQUENCE DESCRIPTION:

        GATGAGTTCGTGTCCGTACAACTGG        25



SEQ ID NO: 5

SEQUENCE LENGTH:  25

SEQUENCE TYPE:  nucleic acid

STRANDEDNESS:  single

TOPOLOGY:  linear

MOLECULE TYPE:  other nucleic acid, synthetic DNA primer

ORIGINAL SOURCE:  λ phage

SEQUENCE DESCRIPTION:

        GGTTATCGAAATCAGCCACAGCGCC        25
```

## Claims

1. An assay method for detecting nucleic acid sequence, which comprises the steps:
   [A] contacting (1) a target single-stranded nucleic acid, (2) a pair of nucleic acid primers having nucleotide sequence complementary to the target nucleic acid and its complementary chain respectively, (3) a fluorescent-labeled nucleic acid probe which has a nucleotide sequence designed so as to be hybridized to the target nucleic acid downstream (at the side of 3'-terminus) from where the nucleic acid primer is hybridized and has a modified 3'-end so that the nucleotide chain is not elongated by a nucleic acid polymerase, and (4) a nucleic acid polymerase having also an exonuclease activity specific to a double-stranded nucleic acid in the presence of four kinds of nucleoside triphosphate which are a substrate for the enzyme, and thereby elongating the said nucleic acid primer chain, and simultaneously hydrolyzing only the fluorescent-labeled nucleic acid probe hybridized to the target nucleic acid by the exonuclease activity of said nucleic acid polymerase,
   [B] denaturating the chain-elongated product of primer to a single-stranded nucleic acid,
   [C] amplifying the target nucleic acid by repeating the above steps [A] and [B] the desired times,
   [D] determining the change in fluorescence polarization resulted by the hydrolysis of the fluorescent-labeled nucleic acid probe.

2. The assay method according to claim 1, wherein the target nucleic acid is DNA, and the nucleic acid moiety of the fluorescent-labeled nucleic acid probe is DNA.

3. The assay method according to claim 1, wherein the target nucleic acid is a cDNA which is derived from RNA by a reverse transcriptase, and the nucleic acid moiety of the fluorescent-labeled nucleic acid probe is DNA.

4. The assay method according to claim 1, wherein the fluorescent-labeled nucleic acid probe is a DNA probe which is labeled with a fluorescein derivative.

11

5. The assay method according to claim 4, wherein the fluorescein derivative is fluorescein isothiocyanate.

6. The assay method according to claim 1, wherein the nucleic acid polymerase is DNA polymerase.

7. The assay method according to claim 6, wherein the DNA polymerase is a DNA polymerase originated from *Thermus aquaticus*.